Europäisches Patentamt

European Patent Office

Office européen des brevets

(19)

(11) **EP 0 906 244 B1**

(12) **FASCICULE DE BREVET EUROPEEN**

(45) Date de publication et mention
de la délivrance du brevet:
**23.01.2002 Bulletin 2002/04**

(21) Numéro de dépôt: **97924068.6**

(22) Date de dépôt: **09.05.1997**

(51) Int Cl.[7]: **C01G 25/00**, B01J 23/10,
B01D 53/94

(86) Numéro de dépôt international:
**PCT/FR97/00829**

(87) Numéro de publication internationale:
**WO 97/43214 (20.11.1997 Gazette 1997/50)**

(54) **COMPOSITION A BASE D'OXYDE DE CERIUM ET D'OXYDE DE ZIRCONIUM, PROCEDE DE PREPARATION ET UTILISATION EN CATALYSE**

ZUSAMMENSETZUNG AUS CERIUMOXID UND ZIRKONIUMOXID, VERFAHREN ZUR HERSTELLUNG UND VERWENDUNG IN KATALYSE

CERIUM OXIDE AND ZIRCONIUM OXIDE BASED COMPOSITION, METHOD OF PREPARATION AND UTILISATION IN CATALYSIS

(84) Etats contractants désignés:
**AT BE DE ES FI FR GB IT SE**

(30) Priorité: **15.05.1996 FR 9606051**

(43) Date de publication de la demande:
**07.04.1999 Bulletin 1999/14**

(73) Titulaire: **RHODIA CHIMIE**
**92408 Courbevoie Cédex (FR)**

(72) Inventeurs:
• **AUBERT, Maryline**
**F-17540 Angliers (FR)**
• **BIRCHEM, Thierry**
**F-75116 Paris (FR)**
• **BLANCHARD, Gilbert**
**F-60330 Lagny-le-Sec (FR)**

(74) Mandataire: **Dubruc, Philippe et al**
**Rhodia Services, Direction de la Propriété**
**Industrielle, 40, rue de la Haie-Coq**
**93306 Aubervilliers Cedex (FR)**

(56) Documents cités:
**EP-A- 0 605 274        EP-A- 0 629 438**

**Description**

La présente invention concerne une composition à base d'oxyde de cérium et d'oxyde de zirconium à haute surface spécifique et à capacité élevée de stockage d'oxygène, son procédé de préparation et son utilisation en catalyse notamment pour la catalyse automobile.

On utilise à l'heure actuelle pour le traitement des gaz d'échappement des moteurs à combustion interne (catalyse postcombustion automobile) des catalyseurs dits multifonctionnels. Par multifonctionnels, on entend les catalyseurs capables d'opérer non seulement l'oxydation en particulier du monoxyde de carbone et des hydrocarbures présents dans les gaz d'échappement mais également la réduction en particulier des oxydes d'azote également présents dans ces gaz (catalyseurs "trois voies"). L'oxyde de zirconium et l'oxyde de cérium apparaissent aujourd'hui comme deux constituants particulièrement importants et intéressants pour ce type de catalyseurs.

Pour être efficaces, ces catalyseurs doivent présenter tout d'abord une surface spécifique importante même à température élevée. En outre, on sait que le cérium permet d'assurer un pouvoir tampon vis à vis des variations de la teneur en oxygène du mélange gazeux à traiter et ainsi d'améliorer les performances du catalyseur à l'égard des trois principaux polluants que sont le CO, HC et les NOx. Ce pouvoir tampon est évalué par la capacité à stocker l'oxygène en milieu oxydant et à le restituer en milieu réducteur. Or, cette capacité de stockage de l'oxygène diminue après une exposition à des températures élevées si bien que le taux de conversion des polluants mentionnés plus haut peut devenir insuffisant.

Il existe de ce fait un besoin en catalyseurs susceptibles de pouvoir être utilisés à température élevée et, pour cela, présentant une grande stabilité de leur surface spécifique combinée, si possible, à une stabilité de leur capacité de stockage de l'oxygène.

L'objet de l'invention est donc la mise au point d'une composition catalytique pouvant répondre à ce besoin.

La composition selon un premier mode de réalisation de l'invention est à base d'un oxyde de cérium et d'un oxyde de zirconium dans une proportion atomique cérium/zirconium d'au moins 1, et elle est caractérisée en ce qu'elle présente après calcination 6 heures à 900°C une surface spécifique d'au moins 35m$^2$/g et une capacité de stockage de l'oxygène mesurée à 400°C d'au moins 1,5ml d'O$_2$/g.

Selon un second mode de réalisation de l'invention, la composition est à base d'un oxyde de cérium, d'un oxyde de zirconium et d'un oxyde d'yttrium dans une proportion atomique cérium/zirconium d'au moins 1 et elle est caractérisée en ce qu'elle présente après calcination 6 heures à 900°C une surface spécifique d'au moins 35m$^2$/g et une capacité de stockage de l'oxygène mesurée à 400°C d'au moins 1,5ml d'O$_2$/g. Selon un troisième mode de réalisation, la composition est à base d'une part d'un oxyde de cérium et d'un oxyde de zirconium, dans une proportion atomique cérium/zirconium d'au moins 1, et d'autre part d'au moins un oxyde choisi parmi l'oxyde de scandium et les oxydes de terres rares à l'exception du cérium et elle est caractérisée en ce qu'elle présente après calcination 6 heures à 900°C une surface spécifique d'au moins 35m$^2$/g et une capacité de stockage de l'oxygène à 400°C d'au moins 1,5ml d'O$_2$/g.

En outre, le procédé de préparation des compositions selon l'invention est du type dans lequel on prépare un mélange en milieu liquide contenant un composé du cérium, un composé du zirconium et, le cas échéant, un composé d'yttrium, de scandium ou de terre rare; on chauffe ledit mélange; on récupère le précipité obtenu et on calcine ce précipité, et il est caractérisé en ce qu'on prépare le mélange précité en utilisant une solution de zirconium qui est telle que la quantité de base nécessaire pour atteindre le point équivalent lors d'un dosage acide-base de cette solution vérifie la condition rapport molaire OH$^-$/Zr ≤1,65.

D'autres caractéristiques, détails et avantages de l'invention apparaîtront encore plus complètement à la lecture de la description qui va suivre, ainsi que des divers exemples concrets mais non limitatifs destinés à l'illustrer.

Pour la suite de la description, on entend par surface spécifique, la surface spécifique B.E.T. déterminée par adsorption d'azote conformément à la norme ASTM D 3663-78 établie à partir de la méthode BRUNAUER - EMMETT-TELLER décrite dans le périodique "The Journal of the American Society, 60, 309 (1938)".

Par terre rare, on entend les éléments du groupe constitué par les éléments de la classification périodique de numéro atomique compris inclusivement entre 57 et 71.

La composition selon l'invention peut se présenter suivant plusieurs modes de réalisation, mais dans tous les cas cette composition est à base d'oxyde de cérium et d'oxyde de zirconium et dans une proportion atomique cérium/zirconium qui est d'au moins 1.

Dans le cas du premier mode de réalisation, la composition peut être constituée essentiellement de cérium et de zirconium. Par "constituée essentiellement" on entend que la composition de l'invention peut présenter ses propriétés de stabilité et notamment une activité catalytique en l'absence de tout autre élément du type oxyde utilisé habituellement en catalyse.

Selon un autre mode de réalisation de l'invention, la composition comprend en outre de l'oxyde dyttrium. Elle peut être constituée essentiellement, au sens du terme donné précédemment, de cérium, d'yttrium et de zirconium.

Selon un troisième mode de réalisation, la composition comprend au moins un oxyde choisi parmi les oxydes de terres rares et l'oxyde de scandium à l'exception de l'oxyde de cérium. Elle peut, là aussi, être constituée essentiellement, au sens du terme donné précédemment, de cérium, de zirconium et du ou des oxydes de terres rares et de scandium.

La terre rare peut être plus particulièrement le lanthane, le néodyme ou le praséodyme. Bien entendu, la composition de l'invention peut comprendre plusieurs oxydes de terres rares ou une combinaison d'un ou de plusieurs oxydes de terres rares avec le scandium.

Dans le cas du troisième mode de réalisation, la composition peut comprendre en outre de l'oxyde d'yttrium.

Les compositions de l'invention peuvent répondre à la formule $Ce_xZr_yM_zO_2$ dans laquelle M représente au moins un élément choisi dans le groupe comprenant l'yttrium, le scandium et les terres rares à l'exception du cérium.

Dans le cas où z=0, x peut varier entre 0,5 et 0,95, plus particulièrement entre 0,5 et 0,9 et encore plus particulièrement entre 0,6 et 0,8, les valeurs des bornes étant incluses et x et y étant liés par la relation x+y=1.

Dans le cas où z n'est pas nul, z présente de préférence une valeur d'au plus 0,3 et qui peut être plus particulièrement comprise entre 0,02 et 0,2, et pour ces valeurs de z le rapport x/y peut être compris entre 1 et 19, plus particulièrement entre 1 et 9 et encore plus particulièrement entre 1,5 et 4, les valeurs des bornes autres que 0 étant incluses et x,y et z étant liés par la relation x+y+z=1.

Les compositions de l'invention présentent une surface spécifique après calcination 6 heures à 900°C sous air d'au moins $35m^2/g$. Cette surface peut être plus particulièrement d'au moins $40m^2/g$. Elle peut être encore plus particulièrement d'au moins $45m^2/g$.

Les compositions de l'invention peuvent aussi présenter des surfaces qui restent encore importantes même après calcination 6 heures à 1000°C. Ces surfaces peuvent être d'au moins $14m^2/g$, plus particulièrement d'au moins $20m^2/g$ et encore plus particulièrement d'au moins $30m^2/g$. La présence d'un élément comme l'yttrium, les terres rares et le scandium tels que décrits plus haut permet d'obtenir les compositions présentant les plus hautes surfaces.

Une autre caractéristique des compositions de l'invention est leur capacité de stockage de l'oxygène. Cette capacité mesurée à 400°C est d'au moins 1,5ml d'$O_2$/g. Elle peut être plus particulièrement d'au moins 1,8ml d'$O_2$/g et encore plus particulièrement d'au moins 2ml d'$O_2$/g. Selon des variantes avantageuses de l'invention, notamment pour les compositions présentant un élément comme l'yttrium, les terres rares et le scandium, cette capacité peut être d'au moins 2,5ml d'$O_2$/g. Les capacités données ci-dessus sont des capacités mesurées sur des produits qui ont été préalablement vieillis 6 heures à 900°C.

Les compositions de l'invention peuvent se présenter avantageusement sous forme d'une solution solide. Les spectres en diffraction X de ces compositions révèlent en effet, au sein de ces dernières, l'existence d'une seule phase homogène. Pour les compositions les plus riches en cérium, cette phase correspond en fait à celle d'un oxyde cérique $CeO_2$ cristallisé et dont les paramètres de mailles sont plus ou moins décalés par rapport à un oxyde cérique pur, traduisant ainsi l'incorporation du zirconium et, le cas échéant, de l'autre élément dans le réseau cristallin de l'oxyde de cérium, et donc l'obtention d'une solution solide vraie.

Le procédé de préparation des compositions de l'invention va maintenant être décrit.

La première étape du procédé selon l'invention consiste à préparer un mélange en milieu liquide, généralement en phase aqueuse, contenant au moins un composé de cérium, au moins un composé de zirconium et, le cas échéant un composé d'yttrium, de scandium ou de terre rare. Ce mélange est préparé en utilisant une solution de zirconium.

Cette solution de zirconium peut provenir de l'attaque acide d'un réactif comprenant du zirconium. Comme réactif approprié, on peut citer le carbonate, l'hydroxyde ou l'oxyde de zirconium. L'attaque peut être faite avec un acide inorganique comme l'acide nitrique, l'acide chlorhydrique ou l'acide sulfurique. L'acide nitrique est l'acide préféré, et on peut mentionner ainsi tout particulièrement l'utilisation d'un nitrate de zirconyle provenant de l'attaque nitrique d'un carbonate de zirconium. Ce peut être aussi un acide organique tel que l'acide acétique ou l'acide citrique.

Selon l'invention, cette solution de zirconium doit présenter la caractéristique suivante. La quantité de base nécessaire pour atteindre le point équivalent lors d'un dosage acide-base de cette solution doit vérifier la condition rapport molaire $OH^-/Zr \leq 1,65$. Plus particulièrement, ce rapport peut être d'au plus 1,5 et encore plus particulièrement d'au plus 1,3. Généralement, la surface spécifique de la composition obtenue a tendance à augmenter lorsque ce rapport décroît.

Le dosage acide-base se fait d'une manière connue. Pour l'effectuer dans des conditions optimales, on peut doser une solution qui a été amenée à une concentration d'environ $3.10^{-2}$ mole par litre exprimée en élément zirconium. On y ajoute sous agitation une solution de soude 1N. Dans ces conditions, la détermination du point équivalent (changement du pH de la solution) se fait d'une manière nette. On exprime ce point équivalent par le rapport molaire $OH^-/Zr$.

A titre de composés du cérium, on peut citer notamment les sels de cérium comme les sels de cérium IV tels que nitrates ou nitrates céri-ammoniacal par exemple, qui conviennent ici particulièrement bien. De préférence, on utilise du nitrate cérique. La solution de sels de cérium IV peut contenir du cérium à l'état céreux mais il est préférable qu'elle contienne au moins 85% de cérium IV. Une solution aqueuse de nitrate cérique peut par exemple être obtenue par réaction de l'acide nitrique sur un oxyde cérique hydraté préparé d'une manière classique par réaction d'une solution d'un sel céreux, par exemple le nitrate céreux, et d'une solution d'ammoniaque en présence d'eau oxygénée. On peut également utiliser une solution de nitrate cérique obtenue selon le procédé d'oxydation électrolytique d'une solution de nitrate céreux tel que décrit dans le document FR-A- 2 570 087, et qui peut constituer une matière première intéressante.

On notera ici que la solution aqueuse de sels de cérium IV peut présenter une certaine acidité libre initiale, par exemple une normalité variant entre 0,1 et 4 N. Selon la présente invention, il est autant possible de mettre en oeuvre une solution initiale de sels de cérium IV présentant effectivement une certaine acidité libre comme mentionné ci-dessus, qu'une solution qui aura été préalablement neutralisée de façon plus ou moins poussée par ajout d'une base, telle que par exemple une solution d'ammoniaque ou encore d'hydroxydes d'alcalins (sodium, potassium,...), mais de préférence une solution d'ammoniaque, de manière à limiter cette acidité. On peut alors, dans ce dernier cas, définir de manière pratique un taux de neutralisation (r) de la solution initiale de cérium par l'équation suivante :

$$r = \frac{n3-n2}{n1}$$

dans laquelle n1 représente le nombre total de moles de Ce IV présentes dans la solution après neutralisation; n2 représente le nombre de moles d'ions OH⁻ effectivement nécessaires pour neutraliser l'acidité libre initiale apportée par la solution aqueuse de sel de cérium IV; et n3 représente le nombre total de moles d'ions OH⁻ apportées par l'addition de la base. Lorsque la variante "neutralisation" est mise en oeuvre, on utilise dans tous les cas une quantité de base qui doit être impérativement inférieure à la quantité de base qui serait nécessaire pour obtenir la précipitation totale de l'espèce hydroxyde $Ce(OH)_4$ (r=4). Dans la pratique, on se limite ainsi à des taux de neutralisation n'excédant pas 1, et de préférence encore n'excédant pas 0,5.

Les composés d'yttrium, de scandium ou de terres rares sont de préférence des composés solubles dans l'eau notamment.

A titre de composés de scandium ou de terres rares utilisables dans le procédé de l'invention, on peut par exemple citer les sels d'acides inorganiques ou organiques, par exemple du type sulfate, nitrate, chlorure ou acétate. On notera que le nitrate convient particulièrement bien. Ces composés peuvent aussi être apportés sous forme de sols. Ces sols peuvent être obtenus par exemple par neutralisation par une base d'un sel de ces composés.

Les quantités de cérium, de zirconium et éventuellement de terres rares, d'yttrium et de scandium présentes dans le mélange doivent correspondre aux proportions stoechiométriques requises pour l'obtention de la composition finale désirée.

Le mélange initial étant ainsi obtenu, on procède ensuite, conformément à la deuxième étape du procédé selon l'invention, à son chauffage.

La température à laquelle est menée ce traitement thermique, aussi appelé thermohydrolyse, peut être comprise entre 80°C et la température critique du milieu réactionnel en particulier entre 80 et 350°C, de préférence entre 90 et 200°C.

Ce traitement peut être conduit, selon les conditions de températures retenues, soit sous pression normale atmosphérique, soit sous pression telle que par exemple la pression de vapeur saturante correspondant à la température du traitement thermique. Lorsque la température de traitement est choisie supérieure à la température de reflux du mélange réactionnel (c'est à dire généralement supérieure à 100°C), par exemple choisie entre 150 et 350°C, on conduit alors l'opération en introduisant le mélange aqueux contenant les espèces précitées dans une enceinte close (réacteur fermé plus couramment appelé autoclave), la pression nécessaire ne résultant alors que du seul chauffage du milieu réactionnel (pression autogène). Dans les conditions de températures données ci-dessus, et en milieux aqueux, on peut ainsi préciser, à titre illustratif, que la pression dans le réacteur fermé varie entre une valeur supérieure à 1 Bar ($10^5$ Pa) et 165 Bar (165. $10^5$ Pa), de préférence entre 5 Bar (5. $10^5$ Pa) et 165 Bar (165. $10^5$ Pa). Il est bien entendu également possible d'exercer une pression extérieure qui s'ajoute alors à celle consécutive au chauffage.

Le chauffage peut être conduit soit sous atmosphère d'air, soit sous atmosphère de gaz inerte, de préférence l'azote.

La durée du traitement n'est pas critique, et peut ainsi varier dans de larges limites, par exemple entre 1 et 48 heures, de préférence entre 2 et 24 heures.

A l'issue de l'étape de chauffage, on récupère un précipité solide qui peut être séparé de son milieu par toute technique classique de séparation solide-liquide telle que par exemple filtration, décantation, essorage ou centrifugation .

Il peut être avantageux d'introduire après l'étape de chauffage, une base telle que par exemple une solution d'ammoniaque, dans le milieu de précipitation. Ceci permet d'augmenter les rendements de récupération en l'espèce précipitée.

Il est aussi possible d'ajouter de la même façon, après l'étape de chauffage, de l'eau oxygénée.

Le produit tel que récupéré peut ensuite être soumis à des lavages à l'eau et/ou à l'ammoniaque, à une température comprise entre la température ambiante et la température d'ébullition. Pour éliminer l'eau résiduelle, le produit lavé peut enfin, éventuellement, être séché, par exemple à l'air, et ceci à une température qui peut varier entre 80 et 300°C, de préférence entre 100 et 150°C, le séchage étant poursuivi jusqu'à l'obtention d'un poids constant.

On notera qu'il est bien entendu possible de répéter une ou plusieurs fois, à l'identique ou non, après récupération du produit et addition éventuelle de la base ou de l'eau oxygénée, une étape de chauffage telle que décrite ci-dessus, en remettant alors le produit en milieu liquide, notamment dans l'eau, et en effectuant par exemple des cycles de traitements thermiques.

Dans une dernière étape du procédé, le précipité récupéré, après éventuellement lavage et/ou séchage, est ensuite calciné. Selon un mode de réalisation particulier, il est possible après le traitement de thermohydrolyse et éventuellement après remise du produit en milieu liquide et un traitement supplémentaire de sécher directement le milieu réactionnel obtenu par atomisation.

La calcination est effectuée à une température comprise généralement entre 200 et 1200°C et de préférence entre 300 et 900°C. Cette température de calcination doit être suffisante pour transformer les précurseurs en oxydes, et elle est aussi choisie en fonction de la température d'utilisation ultérieure de la composition catalytique et en tenant compte du fait que la surface spécifique du produit est d'autant plus faible que la température de calcination mise en oeuvre est plus élevée. La durée de la calcination peut quant à elle varier dans de larges limites, par exemple entre 1 et 24 heures, de préférence entre 4 et 10 heures. La calcination est généralement opérée sous air, mais une calcination menée par exemple sous gaz inerte n'est bien évidemment pas exclue.

Les compositions de l'invention telles que décrites plus haut ou telles qu'obtenues dans les procédés mentionnés précédemment se présentent sous forme de poudres mais elles peuvent éventuellement être mises en forme pour se présenter sous forme de granulés, billes, cylindres ou nids d'abeille de dimensions variables. Ces compositions peuvent être appliquées sur tout support utilisé habituellement dans le domaine de la catalyse, c'est à dire notamment des supports inertes thermiquement. Ce support peut être choisi parmi l'alumine, l'oxyde de titane, l'oxyde de cérium, l'oxyde de zirconium, la silice, les spinelles, les zéolites, les silicates, les phosphates de silicoaluminium cristallins, les phosphates d'aluminium cristallins. Les compositions peuvent aussi être utilisées dans des systèmes catalytiques comprenant un revêtement (wash coat) à propriétés catalytiques et à base de ces compositions, sur un substrat du type par exemple monolithe métallique ou en céramique. Le revêtement peut comporter lui aussi un support du type de ceux mentionnés plus haut. Ce revêtement est obtenu par mélange de la composition avec le support de manière à former une suspension qui peut être ensuite déposée sur le substrat.

Ces systèmes catalytiques et plus particulièrement les compositions de l'invention peuvent trouver de très nombreuses applications. Ils sont ainsi particulièrement bien adaptés à, et donc utilisable dans la catalyse de diverses réactions telles que, par exemple, la déshydratation, l'hydrosulfuration, l'hydrodénitrification, la désulfuration, l'hydrodésulfuration, la déshydrohalogénation, le reformage, le reformage à la vapeur, le craquage, l'hydrocraquage, l'hydrogénation, la déshydrogénation, l'isomérisation, la dismutation, l'oxychloration, la déshydrocyclisation d'hydrocarbures ou autres composés organiques, les réactions d'oxydation et/ou de réduction, la réaction de Claus, le traitement des gaz d'échappement des moteurs à combustion interne, la démétallation, la méthanation, la shift conversion.

Dans le cas de ces utilisations en catalyse, les compositions de l'invention sont employées en combinaison avec des métaux précieux. La nature de ces métaux et les techniques d'incorporation de ceux-ci dans ces compositions sont bien connues de l'homme du métier. Par exemple, les métaux peuvent être le platine, le rhodium, le palladium ou l'iridium, ils peuvent notamment être incorporés aux compositions par imprégnation.

Parmi les utilisations citées, le traitement des gaz d'échappement des moteurs à combustion interne (catalyse post combustion automobile) constitue une application particulièrement intéressante.

De ce fait, l'invention concerne aussi l'utilisation d'une composition ou d'un système catalytique tels que décrits plus haut à la fabrication de catalyseur pour post combustion automobile.

Des exemples vont maintenant être donnés. Les résultats sur les surfaces spécifiques, la capacité de stockage de l'oxygène, les conditions de calcination (température et atmosphère) sont donnés dans les tableaux qui suivent les exemples.

Description du test permettant de quantifier le stockage de l'oxygène

Le pouvoir tampon d'une composition vis-à-vis de l'oxygène est évalué par sa capacité à stocker l'oxygène en milieu oxydant et à le restituer en milieu réducteur. Le test évalue la capacité de la composition à successivement oxyder des pulses de monoxyde de carbone et à consommer des pulses d'oxygène pour réoxyder la composition. La méthode employée est dite alternée.

Le gaz porteur est de l'hélium pur à un débit de 10l/h. Les injections se font par l'intermédiaire de boucle contenant 16ml de gaz. Les pulses de CO sont effectués en utilisant un mélange gazeux contenant 5% de CO dilué dans l'hélium tandis que les pulses d'$O_2$ se font à partir d'un mélange gazeux contenant 2,5% d'$O_2$ dilué dans l'hélium. L'analyse des gaz est effectuée par chromatographie à l'aide d'un détecteur de conductivité thermique.

La quantité d'oxygène consommée permet de déterminer la capacité de stockage d'oxygène. La valeur caractéristique du pouvoir de stockage d'oxygène est exprimée en ml d'oxygène (dans les conditions normales de température et de pression) par gramme de produit introduit et elle est mesurée à 400°C. Les mesures de pouvoir de stockage d'oxygène données dans le tableau qui suit sont faites sur des produits prétraités à 900°C sous air pendant 6 heures dans un four à moufle.

EXEMPLE 1

Cet exemple illustre la préparation d'un oxyde mixte de formule $Ce_{0,62}Zr_{0,38}O_2$.
Dans les proportions stoechiométriques requises pour l'obtention de l'oxyde mixte ci-dessus, on mélange une solution de nitrate cérique et une solution de nitrate de zirconyle. Cette dernière a été obtenue par attaque d'un carbonate à l'aide d'acide nitrique concentré. La solution répond, au sens défini plus haut, à la condition rapport molaire $OH^-/Zr = 0,94$.
La concentration de ce mélange (exprimée en oxyde des différents éléments) est ajustée à 80 g/l. Ce mélange est ensuite porté à 150°C pendant 4 heures.
Une solution d'ammoniaque est ensuite ajoutée au milieu réactionnel de telle sorte que le pH soit supérieur à 8,5. Le milieu réactionnel ainsi obtenu est porté à ébullition pendant 2 heures. Après décantation puis soutirage, on remet en suspension le produit solide et le milieu ainsi obtenu est traité pendant 1 heure à 100°C. Le produit est ensuite filtré puis calciné à la température indiquée dans le tableau des résultats.

EXEMPLE 2

Cet exemple illustre la préparation d'un oxyde mixte de formule $Ce_{0,65}Zr_{0,31}Nd_{0,04}O_2$.
Dans les proportions stoechiométriques requises pour l'obtention de l'oxyde mixte ci-dessus, on mélange une solution de nitrate cérique préneutralisée par ajout de $NH_4OH$ tel que r=- 0,22 (r étant tel que défini précédemment), une solution de nitrate de néodyme et une solution de nitrate de zirconyle qui répond, au sens défini plus haut, à la condition rapport molaire $OH^-/Zr = 1,17$.
Le mode opératoire suivi ensuite est identique à celui de l'exemple 1 jusqu'à l'étape de traitement 1 heure à 100°C. Le milieu réactionnel ainsi obtenu est séché par atomisation, puis calciné à la température indiquée dans le tableau des résultats.

EXEMPLE 3

Cet exemple illustre la préparation d'un oxyde mixte de formule $Ce_{0,645}Zr_{0,30}Y_{0,055}$.
Le mélange des solutions est le même que dans l'exemple 2 aux proportions stoechiométriques près, le nitrate de néodyme étant remplacé par du nitrate d'yttrium.
Le mode opératoire ensuite suivi est identique à celui de l'exemple 2.

EXEMPLE 4

Cet exemple illustre la préparation d'un oxyde mixte de formule $Ce_{0,65}Zr_{0,31}La_{0,04}O_2$;
Le mélange des solutions et le mode opératoire suivi sont les mêmes que dans l'exemple 2, le nitrate de néodyme étant remplacé par du nitrate de lanthane.

EXEMPLE 5

Cet exemple illustre la préparation d'un oxyde mixte de formule $Ce_{0,66}Zr_{0,30}Pr_{0,04}O_2$.
Le mélange des solutions et le mode opératoire suivi sont les mêmes que dans l'exemple 2, le nitrate de néodyme étant remplacé par du nitrate de praséodyme.

EXEMPLE 6

Cet exemple illustre la préparation d'un oxyde mixte de formule $Ce_{0,53}Zr_{0,37}La_{0,10}O_2$.
Dans les proportions stoechiométriques requises pour l'obtention de l'oxyde mixte ci-dessus, on mélange une solution de nitrate cérique, une solution de nitrate de lanthane et une solution de nitrate de zirconyle. Cette dernière répond, au sens défini plus haut, à la condition rapport molaire $OH^-/Zr = 1,17$.
Le mode opératoire suivi ensuite est identique à celui de l'exemple 1.

EXEMPLE 7

Cet exemple illustre la préparation d'un oxyde mixte de formule $Ce_{0,525}Zr_{0,315}Pr_{0,16}O_2$.

Dans les proportions stoechiométriques requises pour l'obtention de l'oxyde mixte ci-dessus, on mélange une solution de nitrate cérique préneutralisée par ajout de $NH_4OH$ tel que r=-0,34, une solution de nitrate de praséodyme et une solution de nitrate de zirconyle qui répond, au sens défini plus haut, à la condition rapport molaire $OH^-/Zr = 1,17$.

Le mode opératoire ensuite suivi est identique à celui de l'exemple 2.

EXEMPLE 8

Cet exemple illustre la préparation d'un oxyde mixte de formule $Ce_{0,535}Zr_{0,373}La_{0,047}Nd_{0,045}O_2$.

Le mode opératoire ensuite suivi est identique à celui de l'exemple 1 mais on utilise une solution de nitrate de zirconyle qui répond, au sens défini plus haut, à la condition rapport molaire $OH^-/Zr = 1,17$.

EXEMPLE 9 COMPARATIF

Cet exemple illustre la préparation selon l'art antérieur d'un oxyde mixte de cérium, de zirconium et d'yttrium de formule $Ce_{0,65}Zr_{0,30}Y_{0,05}O_2$.

Dans les proportions stoechiométriques requises pour l'obtention de l'oxyde mixte ci-dessus, on mélange sous agitation une solution de nitrate cérique et une solution de nitrate de zirconyle, qui répond, au sens défini plus haut, à la condition rapport molaire $OH^-/Zr = 1,80$ et une solution de nitrate d'yttrium.

Le mélange est ensuite traité thermiquement à 150°C pendant 4 heures. A l'issue de ce traitement, on introduit dans la suspension obtenue une solution d'ammoniaque de manière à porter le pH à 9,5, le tout étant ensuite agité pendant 30 minutes pour homogénéisation.

On récupère alors par filtration un précipité qui est ensuite essoré, puis remis en suspension dans de l'eau. Cette suspension est alors chauffée à 100°C pendant 1 heure.

Le produit est à nouveau filtré, puis séché dans une étuve à 120°C et enfin calciné à 900°C pendant 6 heures.

EXEMPLE 10 COMPARATIF

Cet exemple illustre la préparation selon l'art antérieur, par précipitation, d'un oxyde mixte de cérium et de zirconium de formule $Ce_{0,765}Zr_{0,235}O_2$.

Dans les proportions stoechiométriques requises pour l'obtention de l'oxyde mixte ci-dessus, on mélange une solution de nitrate céreux et une solution de nitrate de zirconyle. La concentration en oxyde des éléments est ajustée à 172 g/l.

Ce mélange ainsi obtenu est ajouté en 30 minutes sur une solution contenant de l'ammoniaque, de l'eau et de l'eau oxygénée. Le produit ainsi obtenu est lavé plusieurs fois à l'eau déminéralisée par une série de décantations et éliminations des eaux de lavage. Le produit est ensuite filtré, puis calciné à 900°C pendant 6 heures

EXEMPLE 11

Cet exemple illustre la synthèse d'un oxyde mixte de composition $Ce_{0,657}Zr_{0,306}Pr_{0,037}O_2$ à partir d'une solution de nitrate de zirconyle obtenue par dissolution d'un carbonate de zirconyle dans une solution d'acide nitrique et qui répond, au sens défini plus haut, à la condition rapport molaire $OH^-/Zr = 0,86$.

Dans les proportions stoechiométriques requises pour l'obtention de l'oxyde mixte ci-dessus, on prépare une solution aqueuse contenant du nitrate de cérium IV (non pré-neutralisé), du nitrate de praséodyme et du nitrate de zirconyle, telle que la concentration totale en oxyde du mélange soit de 80 g/l.

Le mélange est ensuite traité thermiquement à 150°C pendant 4 heures dans un autoclave sous agitation constante.

A l'issue de ce traitement, on introduit dans la suspension obtenue de l'ammoniaque de manière à porter le pH à 9. Le tout est ensuite maintenu à 100°C pendant 2 heures.

On élimine les eaux-mères par soutirage. Le produit est ensuite remis en suspension et le pH de la suspension est réajusté à 9 par ajout de la quantité nécessaire d'ammoniaque. Le mélange est maintenu sous agitation pendant une heure à 100°C. A l'issue de cette opération de lavage, le produit est à nouveau filtré, puis séché une nuit dans une étuve à 110°C puis calciné à la température indiquée dans le tableau des résultats.

Tableau 1

| Exemple | S.S.* en m2/g 900°C | S.S.* en m2/g 1000°C | OSC** en mlO$_2$/g |
|---------|---------------------|----------------------|---------------------|
| 1 | 39 | 14 | 2,0 |
| 2 | 43 | 21 | 2.5 |
| 3 | 47 | 18 | 2.1 |
| 4 | 44 | 30 | 2.8 |
| 5 | 40 | 21 | 2.8 |
| 6 | 49 | 38 | 2.1 |
| 7 | 43 | 31 | >2 |
| 8 | 58 | 37 | 2,2 |

Tableau 2

| Exemple | S.S.* en m2/g 900°C | OSC** en mlO$_2$/g |
|---------|---------------------|---------------------|
| 9 comparatif | 39 | 1,1 |
| 10 comparatif | 22 | 1,3 |
| 11 | 40 | 2,8 |

* S.S. : Surface spécifique après calcination sous air 6 heures à la température indiquée

** OSC : Capacité de stockage d'oxygène

**Revendications**

**1.** Composition à base d'un oxyde de cérium et d'un oxyde de zirconium dans une proportion atomique cérium/zirconium d'au moins 1, **caractérisée en ce qu'**elle présente une surface spécifique après calcination 6 heures à 900°C d'au moins 35m$^2$/g et une capacité de stockage de l'oxygène à 400°C d'au moins 1,5ml d'O$_2$/g.

**2.** Composition constituée essentiellement d'un oxyde de cérium et d'un oxyde de zirconium dans une proportion atomique cérium/zirconium d'au moins 1, **caractérisée en ce qu'**elle présente une surface spécifique après calcination 6 heures à 900°C d'au moins 35m$^2$/g et une capacité de stockage de l'oxygène à 400°C d'au moins 1,5ml d'O$_2$/g.

**3.** Composition à base d'un oxyde de cérium, d'un oxyde de zirconium et d'un oxyde d'yttrium dans une proportion atomique cérium/zirconium d'au moins 1, **caractérisée en ce qu'**elle présente une surface spécifique après calcination 6 heures à 900°C d'au moins 35m$^2$/g et une capacité de stockage de l'oxygène à 400°C d'au moins 1,5ml d'O$_2$/g.

**4.** Composition à base d'un oxyde de cérium et d'un oxyde de zirconium, dans une proportion atomique cérium/zirconium d'au moins 1, et d'au moins un oxyde choisi parmi l'oxyde de scandium et les oxydes de terres rares à l'exception du cérium, **caractérisée en ce qu'**elle présente une surface spécifique après calcination 6 heures à 900°C d'au moins 35m$^2$/g et une capacité de stockage de l'oxygène à 400°C d'au moins 1,5ml d'O$_2$/g.

**5.** Composition selon la revendication 4, **caractérisée en ce que** la terre rare est le lanthane, le néodyme ou le praséodyme.

**6.** Composition selon l'une des revendications précédentes, **caractérisée en ce qu'**elle présente une surface spécifique après calcination 6 heures à 900°C d'au moins 40m$^2$/g et plus particulièrement d'au moins 45m$^2$/g.

**7.** Composition selon l'une des revendications précédentes, **caractérisée en ce qu'**elle présente une surface spécifique après calcination 6 heures à 1000°C d'au moins 14m$^2$/g et plus particulièrement d'au moins 20m$^2$/g et encore plus particulièrement d'au moins 30m$^2$/g.

**8.** Composition selon l'une des revendications précédentes, **caractérisée en ce qu'**elle présente une capacité de stockage de l'oxygène à 400°C d'au moins 1,8ml d'O$_2$/g, plus particulièrement d'au moins 2ml d'O$_2$/g.

**9.** Composition selon l'une des revendications précédentes, **caractérisée en ce qu'**elle présente une capacité de stockage de l'oxygène à 400°C d'au moins 2,5ml d'O$_2$/g.

**10.** Composition selon l'une des revendications précédentes, **caractérisée en ce qu'**elle répond à la formule Ce$_x$Zr$_y$M$_z$O$_2$ où M représente au moins un élément choisi dans le groupe comprenant l'yttrium, le scandium et les terres rares et où :

- si z=0, x est compris entre 0,5 et 0,95, plus particulièrement entre 0,5 et 0,9 et encore plus particulièrement entre 0,6 et 0,8, les valeurs des bornes étant incluses et x et y étant liés par la relation x+y=1.
- si z>0, z est compris entre 0 et 0,3 et plus particulièrement entre 0,02 et 0,2, et le rapport x/y est compris entre 1 et 19, plus particulièrement entre 1 et 9 et encore plus particulièrement entre 1,5 et 4, , les valeurs des bornes autres que 0 étant incluses et x,y et z étant liés par la relation x+y+z=1.

**11.** Composition selon l'une des revendications précédentes, **caractérisée en ce qu'**elle se présente sous forme d'une solution solide.

**12.** Procédé de préparation d'une composition selon l'une des revendications précédentes, dans lequel on prépare un mélange en milieu liquide contenant un composé du cérium, un composé du zirconium et, le cas échéant, un composé d'yttrium, de scandium ou de terre rare; on chauffe ledit mélange; on récupère le précipité obtenu et on calcine ce précipité, **caractérisé en ce qu'**on prépare le mélange précité en utilisant une solution de zirconium qui est telle que la quantité de base nécessaire pour atteindre le point équivalent lors d'un dosage acide-base de cette solution vérifie la condition rapport molaire OH$^-$/Zr ≤1,65.

**13.** Procédé selon la revendication 12, **caractérisé en ce qu'**on utilise comme solution de zirconium un nitrate de zirconyle obtenu par attaque nitrique d'un carbonate de zirconium.

**14.** Procédé selon l'une des revendications 13 ou 14, **caractérisé en ce qu'**on utilise une solution de zirconium qui est telle que la quantité de base précitée vérifie la condition rapport molaire OH$^-$/Zr ≤1,5 et encore plus particulièrement OH$^-$/Zr ≤1,3.

**15.** Procédé selon l'une des revendications 12 à 14, **caractérisé en ce qu'**on utilise comme composé de cérium, de scandium et de terres rares un sel de ces éléments, plus particulièrement un nitrate.

**16.** Revêtement à propriétés catalytique, **caractérisé en ce qu'**il comprend une composition selon l'une des revendications 1 à 11 sur un support du type alumine, oxyde de titane, oxyde de cérium, oxyde de zirconium, silice, spinelles, zéolites, silicates, phosphates de silicoaluminium cristallins, phosphates d'aluminium cristallins.

**17.** Système catalytique **caractérisé en ce qu'**il comprend un revêtement à base d'une composition selon l'une des revendications 1 à 11 sur un substrat

**18.** Utilisation d'une composition selon l'une des revendications 1 à 11 ou d'un système catalytique selon la revendication 17 au traitement de gaz d'échappement des moteurs à combustion interne.

**19.** Utilisation d'une composition selon l'une des revendications 1 à 11 ou d'un système catalytique selon la revendication 17 à la fabrication de catalyseurs pour post combustion automobile.

**Patentansprüche**

**1.** Zusammensetzung auf der Basis eines Ceriumoxides und eines Zirkoniumoxides in einem Atomverhältnis Cerium/ Zirkonium von mindestens 1, **dadurch gekennzeichnet, daß** sie eine spezifische Oberfläche nach der Kalzinierung 6 Stunden lang bei 900 °C von mindestens 35 m$^2$/g und eine Kapazität zur Speicherung von Sauerstoff bei 400 °C von mindestens 1,5 ml O$_2$/g aufweist.

**2.** Zusammensetzung, im wesentlichen bestehend aus einem Ceriumoxid und einem Zirkoniumoxid in einem Atomverhältnis Cerium/Zirkonium von mindestens 1, **dadurch gekennzeichnet, daß** sie eine spezifische Oberfläche nach der Kalzinierung 6 Stunden lang bei 900 °C von mindestens 35 m$^2$/g und eine Kapazität zur Speicherung von Sauerstoff bei 400 °C von mindestens 1,5 ml O$_2$/g aufweist.

3. Zusammensetzung auf der Basis eines Ceriumoxides, eines Zirkoniumoxides und eines Yttriumoxides in einem Atomverhältnis Cerium/Zirkonium von mindestens 1, **dadurch gekennzeichnet, daß** sie eine spezifische Oberfläche nach der Kalzinierung 6 Stunden lang bei 900 °C von mindestens 35 m$^2$/g und eine Kapazität zur Speicherung von Sauerstoff bei 400 °C von mindestens 1,5 ml O$_2$/g aufweist.

4. Zusammensetzung auf der Basis eines Ceriumoxides und eines Zirkoniumoxides in einem Atomverhältnis Cerium/ Zirkonium von mindestens 1, und mindestens einem Oxid, ausgewählt unter Scandiumoxid und den Oxiden der Seltenen Erden mit Ausnahme von Cerium, **dadurch gekennzeichnet, daß** sie eine spezifische Oberfläche nach der Kalzinierung 6 Stunden lang bei 900 °C von mindestens 35 m$^2$/g und eine Kapazität zur Speicherung von Sauerstoff bei 400 °C von mindestens 1,5 ml O$_2$/g aufweist.

5. Zusammensetzung nach Anspruch 4, **dadurch gekennzeichnet, daß** die Seltene Erde Lanthan, Neodym oder Praseodym ist.

6. Zusammensetzung nach einem der vorstehenden Ansprüche, **dadurch gekennzeichnet, daß** sie eine spezifische Oberfläche nach der Kalzinierung 6 Stunden lang bei 900 °C von mindestens 40 m$^2$/g und ganz besonders von mindestens 45 m$^2$/g aufweist.

7. Zusammensetzung nach einem der vorstehenden Ansprüche, **dadurch gekennzeichnet, daß** sie eine spezifische Oberfläche nach der Kalzinierung 6 Stunden lang bei 1000 °C von mindestens 14 m$^2$/g, insbesondere von mindestens 20 m$^2$/g und noch spezieller von mindestens 30 m$^2$/g aufweist.

8. Zusammensetzung nach einem der vorstehenden Ansprüche, **dadurch gekennzeichnet, daß** sie eine Kapazität zur Speicherung von Sauerstoff bei 400 °C von mindestens 1,8 ml O$_2$/g, ganz besonders von mindestens 2 ml O$_2$/g aufweist.

9. Zusammensetzung nach einem der vorstehenden Ansprüche, **dadurch gekennzeichnet, daß** sie eine Kapazität zur Speicherung von Sauerstoff bei 400 °C von mindestens 2,5 ml O$_2$/g aufweist.

10. Zusammensetzung nach einem der vorstehenden Ansprüche, **dadurch gekennzeichnet, daß** sie der Formel Ce$_x$Zr$_y$M$_z$O$_2$ entspricht, worin M mindestens ein Element darstellt, gewählt aus der Gruppe, die Yttrium, Scandium und die Seltenen Erden umfaßt, und worin

- wenn z=0 ist, x zwischen 0,5 und 0,95, insbesondere zwischen 0,5 und 0,9 und noch spezieller zwischen 0,6 und 0,8 beträgt, wobei die Grenzwerte eingeschlossen sind und x und y durch die Beziehung x+y=1 verbunden sind;
- wenn z > 0 ist, z zwischen 0 und 0,3 und ganz besonders zwischen 0,02 und 0,2 beträgt und das Verhältnis x/y zwischen 1 und 19, insbesondere zwischen 1 und 9 und noch spezieller zwischen 1,5 und 4 liegt, wobei die Grenzwerte, die sich von 0 unterscheiden, eingeschlossen sind, und x, y und z durch die Beziehung x+y+z=1 verbunden sind.

11. Zusammensetzung nach einem der vorstehenden Ansprüche, **dadurch gekennzeichnet, daß** sie in Form einer festen Lösung vorliegt.

12. Verfahren zur Herstellung einer Zusammensetzung nach einem der vorstehenden Ansprüche, bei dem man eine Mischung im flüssigen Medium herstellt, die eine Verbindung von Cerium, eine Verbindung von Zirkonium und gegebenenfalls eine Verbindung von Yttrium, Scandium oder der Seltenen Erden enthält; die genannte Mischung erhitzt; den erhaltenen Niederschlag sammelt und diesen Niederschlag kalziniert, **dadurch gekennzeichnet, daß** man die oben genannte Mischung unter Verwendung einer Lösung von Zirkonium herstellt, die so beschaffen ist, daß die für das Erreichen des Äquivalenzpunktes notwendige Basismenge bei einer Bestimmung Säure-Base dieser Lösung die Bedingung des molaren Verhältnisses OH$^-$/Zr $\leq$ 1,65 erfüllt.

13. Verfahren nach Anspruch 12, **dadurch gekennzeichnet, daß** man als Zirkonium-Lösung ein Zirkonylnitrat verwendet, erhalten durch Einwirkung von Salpetersäure auf ein Zirkoniumcarbonat.

**14.** Verfahren nach einem der Ansprüche 13 oder 14, **dadurch gekennzeichnet, daß** man eine Zirkonium-Lösung verwendet, die so beschaffen ist, daß die oben genannte Basismenge die Bedingung des molaren Verhältnisses $OH^-/Zr \leq 1{,}5$ und noch spezieller $OH^-/Zr \leq 1{,}3$ erfüllt.

**15.** Verfahren nach einem der Ansprüche 12 bis 14, **dadurch gekennzeichnet, daß** man als Verbindung von Cerium, Scandium und der Seltenen Erden ein Salz dieser Elemente, insbesondere ein-Nitrat verwendet.

**16.** Überzug mit katalytischen Eigenschaften, **dadurch gekennzeichnet, daß** er eine Zusammensetzung nach einem der Ansprüche 1 bis 11 auf einem Träger vom Typ Aluminiumoxid, Titanoxid, Ceriumoxid, Zirkoniumoxid, Siliciumdioxid, Spinell, Zeolithe, Silicate, kristalline Silicoaluminiumphosphate, kristalline Aluminiumphosphate umfaßt.

**17.** Katalytisches System, **dadurch gekennzeichnet, daß** es einen Überzug auf der Basis einer Zusammensetzung nach einem der Ansprüche 1 bis 11 auf einem Substrat umfaßt.

**18.** Verwendung einer Zusammensetzung nach einem der Ansprüche 1 bis 11 oder eines katalytischen Systems nach Anspruch 17 zur Behandlung von Auspuffgasen von Motoren mit innerer Verbrennung.

**19.** Verwendung einer Zusammensetzung nach einem der Ansprüche 1 bis 11 oder eines katalytischen Systems nach Anspruch 17 zur Herstellung von Katalysatoren für die Nachverbrennung bei Kraftfahrzeugen.

## Claims

**1.** A composition based on a cerium oxide and a zirconium oxide in a cerium/zirconium atomic ratio of at least 1, **characterized in that** it has a specific surface area, after calcining for 6 hours at $900°C$, of at least 35 $m^2/g$ and an oxygen storage capacity at $400°C$ of at least 1.5 ml $O_2/g$.

**2.** A composition essentially constituted by a cerium oxide and a zirconium oxide in a cerium/zirconium atomic ratio of at least 1, **characterized in that** it has a specific surface area, after calcining for 6 hours at $900°C$, of at least 35 $m^2/g$ and an oxygen storage capacity at $400°C$ of at least 1.5 ml $O_2/g$.

**3.** A composition based on a cerium oxide, a zirconium oxide and an yttrium oxide in a cerium/zirconium atomic ratio of at least 1, **characterized in that** it has a specific surface area, after calcining for 6 hours at $900°C$, of at least 35 $m^2/g$ and an oxygen storage capacity at $400°C$ of at least 1.5 ml $O_2/g$.

**4.** A composition based on a cerium oxide and a zirconium oxide in a cerium/zirconium atomic ratio of at least 1, and at least one oxide selected from scandium oxide and rare earth oxides with the exception of cerium, **characterized in that** it has a specific surface area, after calcining for 6 hours at $900°C$, of at least 35 $m^2/g$ and an oxygen storage capacity at $400°C$ of at least 1.5 ml $O_2/g$.

**5.** A composition according to claim 4, **characterized in that** the rare earth is lanthanum, neodymium or praseodymium.

**6.** A composition according to any one of the preceding claims, **characterized in that** it has a specific surface area after calcining for 6 hours at $900°C$ of at least 40 $m^2/g$, more particularly at least 45 $m^2/g$.

**7.** A composition according to any one of the preceding claims, **characterized in that** it has a specific surface area, after calcining for 6 hours at $1000°C$, of at least 14 $m^2/g$, more particularly at least 20 $m^2/g$ and still more particularly at least 30 $m^2/g$.

**8.** A composition according to any one of the preceding claims, **characterized in that** it has an oxygen storage capacity at $400°C$ of at least 1.8 ml $O_2/g$, more particularly at least 2 ml $O_2/g$.

**9.** A composition according to any one of the preceding claims, **characterized in that** it has an oxygen storage capacity at $400°C$ of at least 2.5 ml $O_2/g$.

**10.** A composition according to any one of the preceding claims, **characterized in that** it satisfies the formula $Ce_xZr_yM_zO_2$, where M represents at least one element selected from the group formed by yttrium, scandium and the rare earths and in which:

- if z = 0, x is in the range 0.5 to 0.95, more particularly in the range 0.5 to 0.9, still more particularly in the range 0.6 to 0.8, limits included, and x and y are connected by the relationship x + y = 1;
- if z > 0, z is in the range 0 to 0.3, more particularly in the range 0.02 to 0.2, and the ratio x/y is in the range 1 to 19, more particularly in the range 1 to 9, still more particularly in the range 1.5 to 4, limits other than 0 being included, and x, y and z are connected by the relationship x + y + z = 1.

11. A composition according to any one of the preceding claims, **characterized in that** it is in the form of a solid solution.

12. A process for preparing a composition according to any one of the preceding claims, in which a mixture containing a cerium compound, a zirconium compound and, if appropriate, an yttrium, scandium or rare earth compound is prepared in a liquid medium; said mixture is heated; the precipitate obtained is recovered and said precipitate is calcined, **characterized in that** said mixture is prepared using a zirconium solution that is such that the quantity of base necessary to reach the equivalent point during acid-base analysis of said solution satisfies the mole ratio condition $OH^-/Zr \leq 1.65$.

13. A process according to claim 12, **characterized in that** the zirconium solution is a zirconyl nitrate solution obtained by nitric attack on a zirconium carbonate.

14. A process according to claim 13 or claim 14, **characterized in that** a zirconium solution is used that is such that the quantity of said base satisfies the mole ratio condition $OH^-/Zr \leq 1.5$, more particularly $OH^-/Zr \leq 1.3$.

15. A process according to any one of claims 12 to 14, **characterized in that** the cerium, scandium or rare earth compound is a salt of said elements, more particularly a nitrate.

16. A coating with catalytic properties, **characterized in that** it comprises a composition according to any one of claims 1 to 11 on an alumina, titanium oxide, cerium oxide, zirconium oxide, silica, spinel, zeolite, silicate, crystalline silicoaluminium phosphate or crystalline aluminium phosphate type support.

17. A catalytic system, **characterized in that** it comprises a coating based on a composition according to any one of claims 1 to 11 on a substrate.

18. Use of a composition according to any one of claims 1 to 11, or of a catalytic system according to claim 17, for treating exhaust gases from internal combustion engines.

19. Use of a composition according to any one of claims 1 to 11 or of a catalytic system according to claim 17 for the production of catalysts for automobile post combustion.